# EUROPEAN PATENT SPECIFICATION

(11) **EP 2 079 741 B1**
(45) Date of publication and mention of the grant of the patent: **26.11.2014**
(21) Application number: 07820640.6
(22) Date of filing: 27.09.2007
(51) Int. Cl.: C07D 473/16

(54) **PROCESS FOR THE PREPARATION OF ABACAVIR**
VERFAHREN ZUR HERSTELLUNG VON ABACAVIR
PROCÉDÉ DE PRÉPARATION DE L'ABACAVIR

(30) Priority: 28.09.2006 EP 06121459; 28.09.2006 US 847979 P
(43) Date of publication of application: 22.07.2009
(73) Proprietor: ESTEVE QUIMICA, S.A., 08024 Barcelona (ES)
(72) Inventor: MEDRANO RUPÉREZ, Jorge, E-08024 Barcelona (ES); CAMPÓN PARDO, Julio, E-08024 Barcelona (ES); ELIAS RIUS, Laia, E-08024 Barcelona (ES); BERENGUER MAIMÓ, Ramon, E-08024 Barcelona (ES)
(74) Representative: ZBM Patents - Zea, Barlocci & Markvardsen
(86) International application number: PCT/EP2007/060249
(87) International publication number: WO 2008/037760

(56) References cited:
- EP-A- 0 434 450
- WO-A-98/52949
- WO-A-2004/089952
- H. KÖSTER ET AL: TETRAHEDRON, vol. 37, 1981, pages 363-369, XP002416702

## Description

The invention refers to a process for the preparation of an active pharmaceutical ingredient known as abacavir. The process is based on the removal of the protective group of N-2-acyl abacavir using specific basic conditions.

### BACKGROUND ART

Abacavir, is the International Nonproprietary Name (INN) of {(1S,4R)-4-[2-amino-6-(cyclopropylamino)-9H-purin-9-yl]-cyclopent-2-enyl}methanol, and CAS No. 136470-78-5. Abacavir sulfate is a potent selective inhibitor of HIV-1 and HIV-2, and can be used in the treatment of human immunodeficiency virus (HIV) infection.

The structure of abacavir hemisulfate salt corresponds to formula (I):

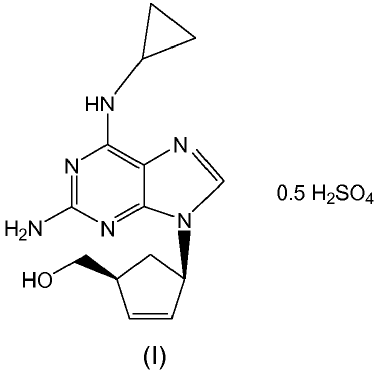

EP 434450-A discloses certain 9-substituted-2-aminopurines including abacavir and its salts, methods for their preparation, and pharmaceutical compositions using these compounds.

Different preparation processes of abacavir are known in the art. In some of them abacavir is obtained starting from an appropriate pyrimidine compound, coupling it with a sugar analogue residue, followed by a cyclisation to form the imidazole ring and a final introduction of the cyclopropylamino group at the 6 position of the purine ring. Pyrimidine compounds which have been identified as being useful as intermediates of said preparation processes include N-2-acylated abacavir intermediates such as N-{6-(cyclopropylamino)-9-[(1R,4S)-4-(hydroxymethyl)cyclopent-2-enyl]-9H-purin-2-yl}acetamide or N-{6-(cyclopropylamino)-9-[(1R,4S)-4-(hydroxymethyl)cyclopent-2-enyl]-9H-purin-2-yl}isobutyramide. The removal of the amino protective group of these compounds using acidic conditions is known in the art. According to Example 28 of EP 434450-A, the amino protective group of the N-{6-(cyclopropylamino)-9-[(1R,4S)-4-(hydroxymethyl)cyclopent-2-enyl]-9H-purin-2-yl}isobutyramide is removed by stirring with 1 N hydrochloric acid for 2 days at room temperature. The abacavir base, after adjusting the pH to 7.0 and evaporation of the solvent, is finally isolated by trituration and chromatography. Then, it is transformed by reaction with an acid to the corresponding salt of abacavir. The main disadvantages of this method are: (i) the use of a strongly corrosive mineral acid to remove the amino protective group; (ii) the need of a high dilution rate; (iii) a long reaction time to complete the reaction; (iv) the need of isolating the free abacavir; and (v) a complicated chromatographic purification process.

Thus, despite the teaching of this prior art document, the research of new deprotection processes of a N-acylated {(1S,4R)-4-[2-amino-6-(cyclopropylamino)-9H-purin-9-yl]-cyclopent-2-enyl}methanol is still an active field, since the industrial exploitation of the known process is difficult, as it has pointed out above. Thus, the provision of a new process for the removal of the amino protective group of a N-acylated {(1S,4R)-4-[2-amino-6-(cyclopropylamino)-9H-purin-9-yl]-cyclopent-2-enyl}methanol is desirable.

### SUMMARY OF THE INVENTION

Inventors have found that the removal of the amino protective group of a N-2-acylated {(1S,4R)-4-[2-amino-6-(cyclopropylamino)-9H-purin-9-yl]-cyclopent-2-enyl}methanol using a base in a mixture of water and alcohol proceeds very fast and the product can be obtained in a high yield and with a high purity since there is no significance formation of by-products compared with the method known in the art.

Thus, the present invention refers to the provison of a process for the preparation of abacavir of formula (I), or pharmaceutically acceptable salts thereof, or solvates thereof, comprising reacting a compound of formula (II) with an inorganic base in a mixture of (C₁-C₆)-alcohol and water, where R is H or a (C₁-C₄)-alkyl radical.

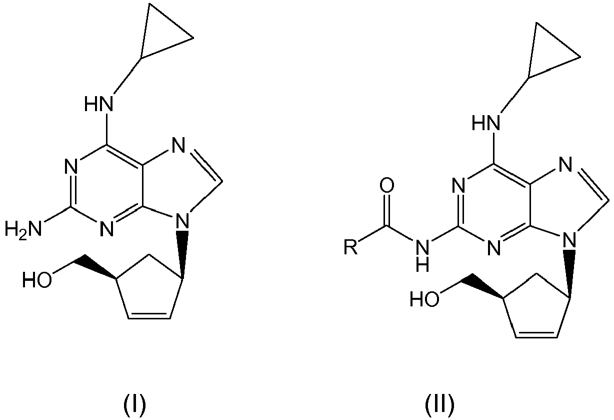

Among the striking advantageous features of the process of the present invention, the following can be mentioned: (i) the hydrolysis carried out in said basic conditions is more efficient; (ii) shorter reaction times are required, since the reaction conditions of the process of the invention allow to perform the hydrolysis at higher temperatures; (iii) lower formation of impurities; in the reaction conditions of the present invention the hydrolysis takes place with a low formation of by-products even at high temperatures, on the contrary, when acidic conditions are used, a fast degradation of the product is observed upon warming ; (iv) the reaction volumes are optimized given that the hydrolysis can be carried out at high concentrations; (vi) it takes place without racemization; (vii) the abacavir or its salts are easyly isolated and purified; and (vii) high yields are obtained.

### DETAILED DESCRIPTION OF THE INVENTION

As described above, abacavir can be obtained by hydrolysis in basic conditions from compound of formula (II) using an inorganic base. In preferred embodiments compounds of formula (II) are those where R is H, methyl or isopropyl. In a more preferred embodiment the compound of formula (II) is N-{6-(cyclopropylamino)-9-[(1R,4S)-4-(hydroxymethyl)cyclopent-2-enyl]-9H-purin-2-yl}isobutyramide (compound of formula (II) where R = isopropyl).

Preferably, the base is an alkaline metal hydroxide such as lithium, sodium or potassium hydroxide. The most preferred alkaline metal hydroxide is sodium hydroxide. Preferably, the amount of inorganic base is comprised between 0.1 and 10 mol of base per mol of starting material of formula (II). More preferably, the amount of base is comprised between 1 and 5 mol of base per mol of starting material.

The hydrolysis is carried out in a mixture of water and an alcohol such as ethanol, n-propanol, isopropanol, n-butanol, isobutanol or t-butanol. Preferably, the solvent system is a mixture of isopropanol and water. Usually the amount of solvent is comprised between 1 and 15 ml/g of starting material. Preferably, between 2 and 10 ml/g. Likewise, the amount of water is usually comprised between 1-15 ml/g of starting material. Preferably between 1 and 10 ml/g.

The reaction is preferably carried out at a temperature comprised between room temperature and the reflux temperature of the solvent used. In a preferred embodiment, the reaction is carried out at a temperature comprised between 50 °C and the reflux temperature of the mixture. Thus, it is advantageous since surprisingly the reaction time is tremendously reduced at these temperatures while no significance by-products formation is observed. In a more preferred embodiment, the reaction is carried out at the reflux temperature of the mixture.

The abacavir can be isolated from the reaction medium as a pharmaceutically acceptable salt, preferably the hemisulfate salt, by separating the aqueous phase and precipitating the salt of abacavir from the organic phase by addition of the appropriate amount of the corresponding pharmaceutically acceptable acid. Optionally, a second solvent can be added before separation of the aqueous phase. Examples of suitable solvents include (C₂-C₈) aliphatic ethers such as ethyl ether, isopropyl ether, tert-butylmethyl ether, di-n-butyl ether or tetrahydrofuran, (C₆-C₈)-aromatic hydrocarbons such as toluene or xylene, or chlorine-containing solvents such as chloroform or methylene chloride. Optionally, the organic phase can be washed with aqueous sodium hydroxide or with an aqueous solution of another inorganic base before the addition of the pharmaceutically acceptable acid. Higher yields may be obtained when the salt of abacavir is isolated from a solvent in an anhydrous medium. For instance, the water can be removed by azeotropic distillation or by evaporation to dryness and then adding the appropriate solvent to precipitate the salt of abacavir.

The hemisulfate salt of {(1S,4R)-4-[2-amino-6-(cyclopropylamino)-9H-purin-9-yl]-cyclopent-2-enyl}methanol means the salt formed between {(1S,4R)-{4-[2-amino-6-(cyclopropylamino)-9H-purin-9-yl]-cyclopent-2-enyl}methanol and sulfuric acid in a stoichiometric ratio of 2:1.

Alternatively, abacavir can be isolated from the reaction medium as a free base by crystallization. A change of solvent may be carried out to perform the crystallization. Suitable crystallization solvent system is, for instance, (C₂-C₆)-alcohols such as ethanol, n-propanol, isopropanol, n-butanol, isobutanol or tert-butanol, (C₃-C₉)-ketones such as acetone, methylisobutylketone, or methylethylketone, (C₂-C₈) aliphatic ethers such as ethyl ether, isopropyl ether, tert-butylmethyl ether, di-n-butyl ether or tetrahydrofuran, (C₂-C₁₀)-esters such as ethyl acetate, acetonitrile, or mixtures thereof. Preferred solvent systems are acetone, acetonitrile, ethyl acetate, isopropanol or mixtures of isopropanol/tert-butyl methyl ether. Optionally, the organic phase can be washed with aqueous sodium hydroxide or with an aqueous solution of other inorganic base before crystallizing the abacavir as free base.

Abacavir can also be isolated from the reaction medium as a free base by optionally adding a solvent selected from (C₂-C₈)-aliphatic ethers and (C₆-C₈)-aromatic hydrocarbons, separating the aqueous phase, optionally removing the remaining water, and crystallyzing the abacavir of formula (I) as free base in an appropriate solvent system. Preferably, the crystallizing solvent system is selected from those mentioned above. Optionally, the organic phase can be washed with aqueous sodium hydroxide or with an aqueous solution of other inorganic base before crystallizing the abacavir as free base.

When a pharmaceutically acceptable salt is desired, it can also be obtained from the abacavir base by treatment with the corresponding acid. A preferred salt is the hemisulfate salt of abacavir.

The most adequate conditions for carrying out said process vary depending on the parameters considered by an expert in the art, such as, for example, the concentration of the reaction mixture, the temperature, the solvent used during the reaction and the isolation of the product, and the like. These can be readily determined by said skilled person in the art with the help of the teachings of the examples given in this description.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples are provided by way of illustration.

### EXAMPLES

### Example 1: Preparation of abacavir hemisulfate

N-{6-(cyclopropylamino)-9-[(1R,4S)-4-(hydroxymethyl)cyclopent-2-enyl]-9H-purin-2-yl}isobutyramide (6.56 g, 18.40 mmol) was slurried in a mixture of isopropanol (32.8 ml) and 10% solution of NaOH (36.1 ml, 92.0 mmol). The mixture was refluxed for 1 h. The resulting solution was cooled to 20-25 °C and tert-butyl methyl ether (32.8 ml) was added. The layers were separated and H₂SO₄ 96% (0.61 ml, 11.03 mmol) was added dropwise to the organic layer. This mixture was cooled to 0-5 °C and the resulting slurry filtered off. The solid was dried under vacuum at 40 °C. Abacavir hemisulfate (5.98 g, 97%) was obtained as a white powder.

### Example 2: Preparation of abacavir hemisulfate

N-{6-(cyclopropylamino)-9-[(1R,4S)-4-(hydroxymethyl)cyclopent-2-enyl]-9H-purin-2-yl}isobutyramide (6.56 g, 18.40 mmol) was slurried in a mixture of isopropanol (32.8 ml) and 10% solution of NaOH (36.1 ml, 92.0 mmol). The mixture was refluxed for 1 h. The resulting solution was cooled to 20-25 °C and toluene (32.8 ml) was added. The layers were separated and H₂SO₄ 96% (0.61 ml, 11.03 mmol) was added dropwise to the organic layer. This mixture was cooled to 0-5 °C and the resulting slurry filtered off. The solid was dried under vacuum at 40 °C. Abacavir hemisulfate (5.42 g, 88%) was obtained as a white powder.

### Example 3: Preparation of abacavir hemisulfate

To a solution of N-{6-(cyclopropylamino)-9-[(1R,4S)-4-(hydroxymethyl) cyclopent-2-enyl]-9H-purin-2-yl}isobutyramide (1.0 g, 2.80 mmol) in isopropanol (10 ml) a 10% solution of NaOH (5.5 ml, 14.03 mmol) was added. The mixture was refluxed for 1 h. The resulting solution was cooled to 20-25 °C and the aqueous layer was separated. H₂SO₄ 96% (0.07 ml, 1.22 mmol) was added dropwise to the organic layer. The mixture was concentrated to half volume and the salts were filtered off. To the obtained solution, H₂SO₄ 96% (0.07 ml, 1.22 mmol) was added dropwise and cooled to 0-5 °C. The solid was filtered off and dried under vacuum at 40 °C. Abacavir hemisulfate (0.56 g, 60%) was obtained as a white powder.

### Example 4: Preparation of abacavir hemisulfate

N-{6-(cyclopropylamino)-9-[(1R,4S)-4-(hydroxymethyl)cyclopent-2-enyl]-9H-purin-2-yl}isobutyramide (5.0 g, 14.03 mmol) was slurried in a mixture of isopropanol (25 ml) and 10% solution of NaOH (27.5 ml, 70.1 mmol). The mixture was refluxed for 1 h. The resulting solution was cooled to 20-25 °C and the aqueous solution was discarded. The organic layer was concentrated to dryness. isopropanol (10 ml) was added and further concentrated to dryness two times. To this residue, isopropanol (25 ml) was added and the salts were filtered off. To the obtained solution, H₂SO₄ 96% (0.39 ml, 7.0 mmol) was added dropwise. This mixture was cooled to 0-5 °C and the resulting slurry filtered off. The solid was dried under vacuum at 40 °C. Abacavir hemisulfate (3.7 g, 79%) was obtained as a white powder.

### Example 5: Preparation of abacavir hemisulfate

A mixture of N-{6-(cyclopropylamino)-9-[(1R,4S)-4-(hydroxymethyl)cyclopent-2-enyl]-9H-purin-2-yl}isobutyramide (10 g, 28 mmol), isopropanol (100 ml) and 10% solution of NaOH (16.8 ml, 42 mmol) was refluxed for 1 h. The resulting solution was cooled to 20-25 °C and washed several times with 25% solution of NaOH (10 ml). The wet organic layer was neutralized to pH 7.0-7.5 with 17% hydrochloric acid and it was concentrated to dryness under vacuum. The residue was taken in isopropanol (100 ml) and the salts were filtered off. To the filtrate, H₂SO₄ 96% (0.78 ml, 14.0 mmol) was added dropwise. This mixture was cooled to 0-5 °C and the precipitated was filtered and dried under vacuum at 40 °C to afford 15.0 g (80%) of abacavir hemisulfate as a white powder.

### Example 6: Preparation of abacavir

N-{6-(cyclopropylamino)-9-[(1R,4S)-4-(hydroxymethyl)cyclopent-2-enyl]-9H-purin-2-yl}isobutyramide (1.0 g, 2.80 mmol) was slurried in a mixture of isopropanol (2 ml) and 10% solution of NaOH (1.1 ml, 2.80 mmol). The mixture was refluxed for 1 h. The resulting solution was cooled to 20-25 °C and tert-butyl methyl ether (2 ml) was added. The aqueous layer was discarded, the organic phase was cooled to 0-5 °C and the resulting slurry filtered off. The solid was dried under vacuum at 40 °C. Abacavir (0.62 g, 77%) was obtained as a white powder.

### Example 7: Preparation of abacavir

N-{6-(cyclopropylamino)-9-[(1R,4S)-4-(hydroxymethyl)cyclopent-2-enyl]-9H-purin-2-yl}isobutyramide (1.25 g, 3.51 mmol) was slurried in a mixture of isopropanol (2.5 ml) and 10% solution of NaOH (1.37 ml, 3.51 mmol). The mixture was refluxed for 1 h and concentrated to dryness. The residue was crystallized in acetone. Abacavir (0.47 g, 47%) was obtained as a white powder.

### Example 8: Preparation of abacavir

N-{6-(cyclopropylamino)-9-[(1R,4S)-4-(hydroxymethyl)cyclopent-2-enyl]-9H-purin-2-yl}isobutyramide (1.25 g, 3.51 mmol) was slurried in a mixture of isopropanol (2.5 ml) and 10% solution of NaOH (1.37 ml, 3.51 mmol). The mixture was refluxed for 1 h and concentrated to dryness. The residue was crystallized in acetonitrile. Abacavir (0.43 g, 43%) was obtained as a white powder.

### Example 9: Preparation of abacavir

A mixture of N-{6-(cyclopropylamino)-9-[(1R,4S)-4-(hydroxymethyl)cyclopent-2-enyl]-9H-purin-2-yl}isobutyramide (10 g, 28 mmol), isopropanol (100 ml) and 10% solution of NaOH (16.8 ml, 42 mmol) was refluxed for 1 h. The resulting solution was cooled to 20-25 °C and washed several times with 25% solution of NaOH (10 ml). The wet organic layer was neutralized to pH 7.0-7.5 with 17% hydrochloric acid and it was concentrated to dryness under vacuum. The residue was crystallized in ethyl acetate (150 ml) to afford abacavir (7.2 g, 90%).

### Example 10: Preparation of abacavir

A mixture of N-{6-(cyclopropylamino)-9-[(1R,4S)-4-(hydroxymethyl)cyclopent-2-enyl]-9H-purin-2-yl}isobutyramide (10 g, 28 mmol), isopropanol (100 ml) and 10% solution of NaOH (16.8 ml, 42 mmol) was refluxed for 1 h. The resulting solution was cooled to 20-25 °C and washed several times with 25% solution of NaOH (10 ml). The wet organic layer was neutralized to pH 7.0-7.5 with 17% hydrochloric acid and it was concentrated to dryness under vacuum. The residue was crystallized in acetone (300 ml) to afford abacavir (7.0 g, 88%).

## Claims

1. A process for the preparation of abacavir of formula (I), or a pharmaceutically acceptable salt thereof, or a solvate thereof, comprising reacting a compound of formula (II) with an inorganic base in a mixture of a (C₁-C₆)-alcohol and water, wherein R is H or a (C₁-C₄)-alkyl radical.

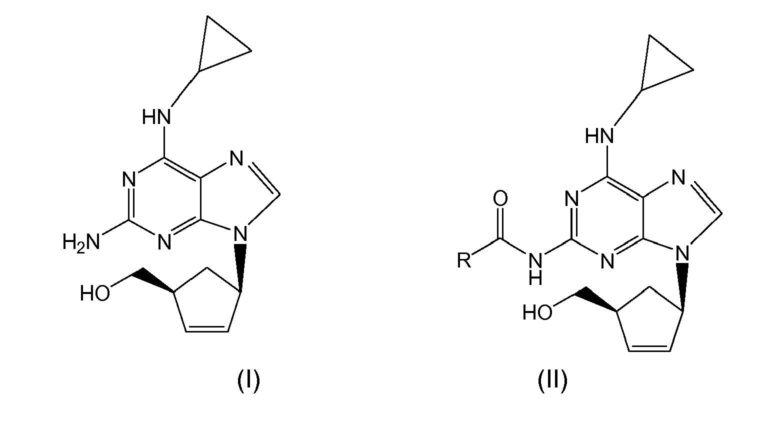

2. The preparation process according to claim 1, wherein R is isopropyl.

3. The preparation process according to any of the claims 1-2, wherein the base is an alkaline metal hydroxide.

4. The preparation process according to claim 3, wherein the alkaline metal hydroxide is sodium hydroxide.

5. The preparation process according to any of the claims 1-4, wherein the alcohol is isopropanol.

6. The preparation process according to any of the claims 1-5, wherein the reaction is carried out at a temperature comprised between 50 °C and the reflux temperature of the mixture.

7. The preparation process according to any of the claims 1-6, wherein abacavir (I) is isolated as a salt and the isolation process comprises: (i) optionally adding a solvent selected from the group consisting of (C₂-C₈)-aliphatic ethers, (C₆-C₈)-aromatic hydrocarbons and chlorine-containing solvents; (ii) separating the aqueous phase; (iii) optionally, carrying out at least one wash with an aqueous solution of an inorganic base; (iv) optionally removing the remaining water; and (v) precipitating the pharmaceutically acceptable salt from the organic phase by addition of the appropriate amount of the corresponding pharmaceutically acceptable acid.

8. The preparation process according to claim 7, wherein the salt of the abacavir (I) is the hemisulfate salt.

9. The preparation process according to any of the claims 1-6, wherein the abacavir of formula (I) is isolated as a free base and the isolation process comprises crystallizing the abacavir (I) as free base in an appropriate solvent system; and if desired, treating the compound obtained with a pharmaceutically acceptable acid to form the corresponding salt.

10. The preparation process according to any of the claims 1-6, wherein the abacavir (I) is isolated as free base and the isolation process comprises: (i) optionally adding a solvent selected from the group consisting of (C₂-C₈)-aliphatic ethers and (C₆-C₈)-aromatic hydrocarbons; (ii) separating the aqueous phase; (iii) optionally, carrying out at least one wash with an aqueous solution of an inorganic base; (iv) optionally removing the remaining water; and (v) crystallyzing the abacavir (I) as free base in an appropriate solvent system; and if desired, treating the compound obtained in step (v) with a pharmaceutically acceptable acid to form the corresponding salt.

11. The preparation process according to any of the claims 9-10, wherein the crystallization solvent system of the abacavir free base is selected from the group consisting of acetone, acetonitrile, ethyl acetate, isopropanol and mixtures of isopropanol/tert-butyl methyl ether.

## Patentansprüche

1. Ein Verfahren zur Herstellung von Abacavir der Formel (I), oder eines pharmazeutisch akzeptablen Salzes davon, oder eines Solvats davon, umfassend die Reaktion einer Verbindung der Formel (II) mit einer organischen Base in einer Mischung aus (C₁-C₆)-Alkohol und Wasser, wobei R H oder ein (C₁-C₄)-Alkylrest ist.

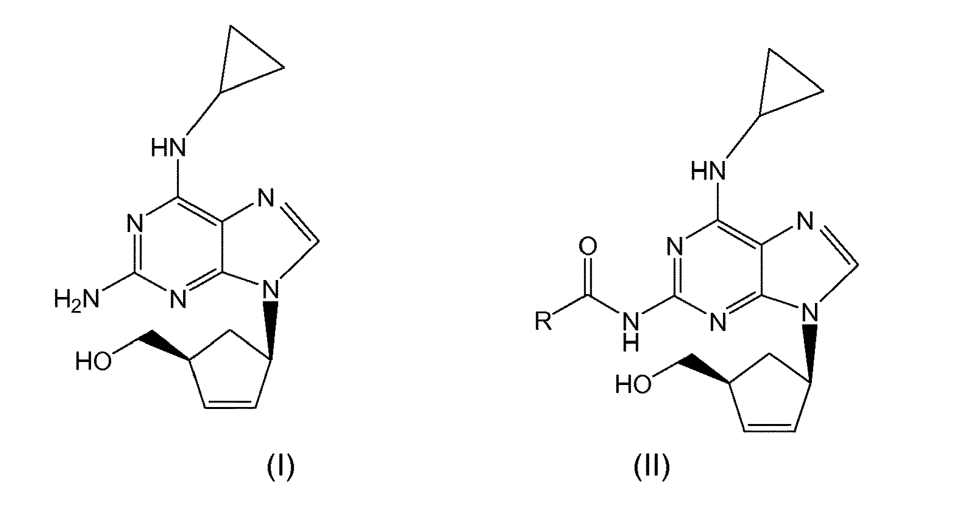

2. Das Herstellungsverfahren nach Anspruch 1, wobei R Isopropyl ist.

3. Das Herstellungsverfahren nach einem der Ansprüche 1-2, wobei die Base ein Alkalimetallhydroxid ist.

4. Das Herstellungsverfahren nach Anspruch 3, wobei das Alkalimetallhydroxid Natriumhydroxid ist.

5. Das Herstellungsverfahren nach einem der Ansprüche 1-4, wobei der Alkohol Isopropanol ist.

6. Das Herstellungsverfahren nach einem der Ansprüche 1-5, wobei die Reaktion bei einer Temperatur durchgeführt wird, die bei zwischen 50 °C und der Rücklauftemperatur der Mischung liegt.

7. Das Herstellungsverfahren nach einem der Ansprüche 1-6, wobei das Abacavir (I) als ein Salz isoliert wird und das Isolierungsverfahren folgendes umfasst: (i) die fakultative Zugabe eines Lösungsmittels, das ausgewählt aus der Gruppe bestehend aus (C₂-C₈)-aliphatischen Ethern, (C₆-C₈)-aromatischen Kohlenwasserstoffen und chlorhaltigen Lösungsmitteln ist; (ii) die Abtrennung der wässrigen Phase; (iii) die fakultative Durchführung von mindestens einem Waschverfahren mit einer wässrigen Lösung einer anorganischen Base; (iv) das fakultative Entfernen des übrigen Wassers; und (v) das Abscheiden des pharmazeutisch akzeptablen Salzes aus der organischen Phase durch die Zugabe einer angemessenen Menge der entsprechenden pharmazeutisch akzeptablen Säure.

8. Das Herstellungsverfahren nach Anspruch 7, wobei das Salz des Abacavirs (I) das Hemisulfatsalz ist.

9. Das Herstellungsverfahren nach einem der Ansprüche 1-6, wobei das Abacavir der Formel (I) als eine freie Base isoliert wird und das Isolierungsverfahren die Kristallisation des Abacavirs (I) als freie Base in einem geeigneten Lösungsmittelsystem umfasst; und, falls gewünscht, die Behandlung der erhaltenen Verbindung mit einer pharmazeutisch akzeptablen Säure, um das entsprechende Salz zu erzeugen.

10. Das Herstellungsverfahren nach einem der Ansprüche 1-6, wobei das Abacavir (I) als freie Base isoliert wird und das Isolierungsverfahren folgendes umfasst: (i) die fakultative Zugabe eines Lösungsmittels, das ausgewählt aus der Gruppe bestehend aus (C₂-C₈)-aliphatischen Ethern und (C₆-C₈)-aromatischen Kohlenwasserstoffen ist; (ii) die Abtrennung der wässrigen Phase; (iii) die fakultative Durchführung von mindestens einem Waschverfahren mit einer wässrigen Lösung einer anorganischen Base; (iv) das fakultative Entfernen des übrigen Wassers; und (v) die Kristallisation des Abacavirs (I) als freie Base in einem geeigneten Lösungsmittelsystem; und, falls gewünscht, die Behandlung der im Schritt (v) erhaltenen Verbindung mit einer pharmazeutisch akzeptablen Säure, um das entsprechende Salz zu erzeugen.

11. Das Herstellungsverfahren nach einem der Ansprüche 9-10, wobei das Kristallisationslösungsmittelsystem des Abacavirs als freie Base ausgewählt aus der Gruppe bestehend aus Aceton, Acetonitril, Ethylacetat, Isopropanol und Mischungen aus Isopropanol/tert-Butylmethylether ist.

## Revendications

1. Un procédé de préparation de l'abacavir de formule (I), ou d'un sel pharmaceutiquement acceptable de celui-ci, ou d'un solvate de celui-ci, comprenant la réaction d'un composé de formule (II) avec une base inorganique dans un mélange d'un alcool en (C₁-C₆) et de l'eau, dans laquelle R est H ou un radical alkyle en (C₁-C₄).

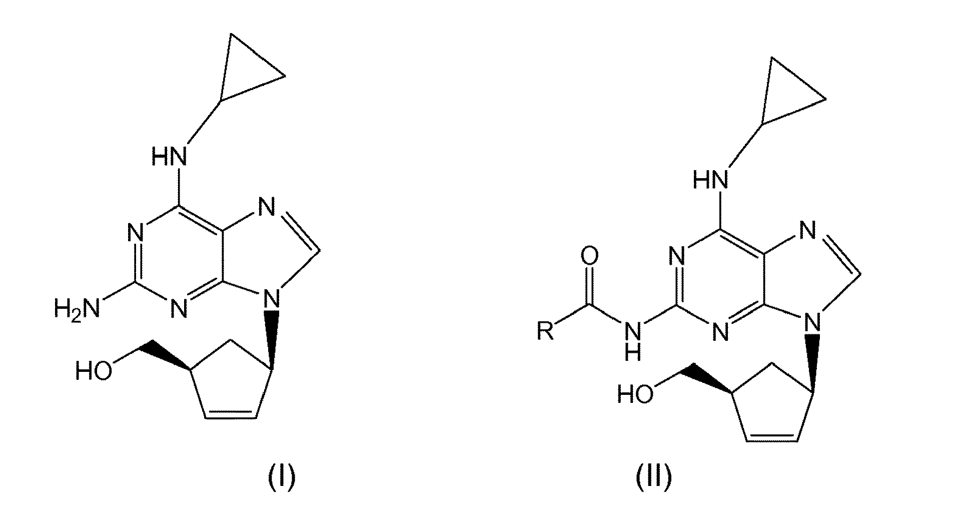

2. Le procédé de préparation selon la revendication 1, dans lequel R est l'isopropyle.

3. Le procédé de préparation selon l'une quelconque des revendications 1-2, dans lequel la base est un hydroxyde de métal alcalin.

4. Le procédé de préparation selon la revendication 3, dans lequel l'hydroxyde de métal alcalin est l'hydroxyde de sodium.

5. Le procédé de préparation selon l'une quelconque des revendications 1-4, dans lequel l'alcool est l'isopropanol.

6. Le procédé de préparation selon l'une quelconque des revendications 1-5, dans lequel la réaction est effectuée à une température comprise entre 50 °C et la température de reflux du mélange.

7. Le procédé de préparation selon l'une quelconque des revendications 1-6, dans lequel l'abacavir (I) est isolé en tant qu'un sel et dans lequel le procédé d'isolation comprend: (i) facultativement ajouter un solvant choisi dans le groupe constitué des éthers aliphatiques en (C₂-C₈), des hydrocarbures aromatiques en (C₆-C₈) et des solvants contenant du chlore; (ii) séparer la phase aqueuse; (iii) facultativement, effectuer au moins un lavage avec une solution aqueuse d'une base inorganique; (iv) facultativement retirer l'eau restante; et (v) précipiter le sel pharmaceutiquement acceptable de la phase organique moyennant l'addition d'une quantité appropriée de l'acide pharmaceutiquement acceptable correspondant.

8. Le procédé de préparation selon la revendication 7, dans lequel le sel de l'abacavir (I) est le sel hémisulfate.

9. Le procédé de préparation selon l'une quelconque des revendications 1-6, dans lequel l'abacavir de formule (I) est isolé en tant qu'une base livre et le procédé d'isolation comprend la cristallisation de l'abacavir (I) en tant qu'une base livre dans un système de solvant approprié; et, si désiré, traiter le composé obtenu avec un acide pharmaceutiquement acceptable afin de former le sel correspondant.

10. Le procédé de préparation selon l'une quelconque des revendications 1-6, dans lequel l'abacavir (I) est isolé en tant qu'une base livre et dans lequel le procédé d'isolation comprend: (i) facultativement, ajouter un solvant choisi dans le groupe constitué des éthers aliphatiques en (C₂-C₈) et des hydrocarbures aromatiques en (C₆-C₈); (ii) séparer la phase aqueuse; (iii) facultativement, effectuer au moins un lavage avec une solution aqueuse d'une base inorganique; (iv) facultativement retirer l'eau restante; et (v) cristalliser l'abacavir (I) en tant qu'une base livre dans un système de solvant approprié; et, si désiré, traiter le composé obtenu dans l'étape (v) avec un acide pharmaceutiquement acceptable afin de former le sel correspondant.

11. Le procédé de préparation selon l'une quelconque des revendications 9-10, dans lequel le système de solvant de cristallisation de la base livre d'abacavir est choisi dans le groupe constitué de l'acétone, de l'acétonitrile, de l'acétate d'éthyle, de l'isopropanol et des mélanges d'isopropanol/méthyl tert-butyl éther.
